(19) Europäisches Patentamt
European Patent Office
Office européen des brevets

(11) Publication number: **0 497 274 A2**

## EUROPEAN PATENT APPLICATION

(21) Application number: **92101350.4**

(22) Date of filing: **28.01.92**

(51) Int. Cl.⁵: **C12N 5/08**, A61K 35/14, A61K 37/02, //(A61K37/02, 35:14)

(30) Priority: **31.01.91 US 648676**

(43) Date of publication of application: **05.08.92 Bulletin 92/32**

(84) Designated Contracting States: **CH DE FR GB IT LI NL SE**

(71) Applicant: **TERUMO KABUSHIKI KAISHA No. 44-1, Hatagaya 2-chome, Shibuya-ku Tokyo 151(JP)**

(72) Inventor: **Leung, Kam H. 709 Marshall Road Brookhaven, Pennsylvania 19015(US)**

(74) Representative: **von Kreisler, Alek, Dipl.-Chem. et al Patentanwälte Von Kreisler-Selting-Werner, Deichmannhaus am Hauptbahnhof W-5000 Köln 1(DE)**

(54) Generation of adherent LAK cells with interleukin-2 and interleukin-4.

(57) Peripheral blood mononuclear cells (PBMC) are treated to deplete monocytes and the remaining cells are cultured in a medium containing interleukin-2 (IL-2) without interleukin-4 (IL-4). Nonadherent cells are removed and discarded, and adherent cells are further cultured in a medium of IL-2 and IL-4 which expands the A-LAK cell population of the PBMC. The expanded, enriched A-LAK cells can be placed in a pharmaceutically acceptable carrier and administered to a mammal with IL-2 to treat a tumor.

EP 0 497 274 A2

CROSS-REFERENCE

The following application, filed concurrently herewith, is incorporated herein by reference: Kam H. Leung, "Generation of CD4[+] Helper T Cells with Interleukin 2 and Interleukin 4 from L-phenylalanine Methyl Ester Treated Human Peripheral Blood Cells", Serial No.                    .

BACKGROUND OF THE INVENTION

Natural killer (NK) cells and lymphokine-activated killer (LAX) cells have been implicated in immunosurveillance against tumor cells (Barlozzani et al. (1983) J. Immunol., 131, 1024; Rayner et al. (1985) Cancer, 55:1327. It has been reported that the systemic administration of autologous LAK cells to patients with advanced cancer is beneficial (Mule et al. (1986) Cancer Res., 46: 676). This process is tedious in that large numbers of cells are needed for each patient. Also, the treatment of cancer patients using LAK and interleukin-2 (IL-2) is accompanied by substantial toxicities (Rosenberg et al. (1987) N. Engl. J. Med., 316: 889).

It has been shown that monocytes interfere with the activation of LAK activity by IL-2 (Rosenberg et al. (1987) N. Engl. J. Med., 316: 889) . L-Leucine methyl ester (LME) and L-phenylalanine methyl ester (PME) were shown to remove monocytes from human peripheral blood mononuclear cells (PBMC) (Thiele and Lipsky (1985) J. shown to remove monocytes from human peripheral blood mononuclear cells (PBMC) (Thiele and Lipsky (1985) J. Immunol., 134: 786; Hoyer et al. (1986) Cancer Res., 46: 2834). However, LME also depleted NK activity and NK cells (Thiele and Lipsky (1985) J. Immunol., 134: 786; Hoyer et al. (1986) Cancer Res., 46: 2834). We have shown that depletion of monocytes by PME allows generation of LAK cells by IL-2 at cell densities of $5\times10^6$/mL or higher (Leung (1987) Lymphokine Research, 6, Abstract #1718; U.S. patent 4,849,329 to Leung and Rinehart.

It has been reported that adherent LAK (A-LAK) cells are generated by adherence to plastic of 24-hr IL-2-activated, monocyte-depleted PBMC (Melder et al. (1988) Cancer Res., 48, 346). These cells are highly proliferative and cytotoxic and are enriched for LAK cells (Leu19[+], LAK phenotype). There are several potential important advantages of A-LAK cells for adoptive immunotherapy in humans. Since A-LAK cells are enriched for LAK cells with greater antitumor activity, A-LAK cells may be effective using fewer cells than conventional LAK cellular therapy. Moreover, less IL-2 may be needed for co-administration with A-LAK cells to patients, thereby reducing the toxicity of the therapy.

In previous reports, monocytes were removed by their adherence to nylon-wool columns or by centrifugal elutriation in order to generate A-LAK cells (Melder et al. (1988) Cancer Res., 48: 346). These procedures for monocyte removal are tedious and complicated. Some LAK cell precursors may also adhere to the nylon-wool columns. Therefore, we have employed PME (5 mM) as a single step for monocyte depletion. We were able to generate A-LAK from PME-treated cells (copending, coassigned U.S. patent application Serial No. 07/384134, filed 7/21/89). Partial depletion of monocytes using lower concentrations (1 to 2.5 mM) of PME allowed more A-LAK cell expansion than did complete monocyte depletion using higher concentrations of PME. Our results suggest that too many monocytes (>10%) in the PBMC population hindered A-LAK generation and a few monocytes (<10%) enhanced A-LAK generation.

IL-2 is a potent stimulator of NK cells and T cells. On the other hand, IL-4 was first described as a B cell growth factor (Howard et al. (1982) J. Exp. Med. 155: 914). IL-4 is considered as a lymphokine with multiple effects on B cells, T cells, NK cells, and monocytes (Widmer et al. (1987) J. Exp. Med. 166: 1447; Mitchell et al. (1989) J. Immunol. 142: 1548; Spits et al. (1987) J. Immunol. 139: 1142; Nagler et al. (1988) J. Immunol. 141: 2349; te Velde et al. (1989) Agents and Actions 26: 1; Spits et al. (1988) J. Immunol. 141: 29; Spits et al. (1988) J. Immunol. 141: 29; Brooks and Rees, (1988) Clin. Exp. Immunol. 74: 162; Kawakami et al. (1989) J. Immunol. 142: 3452). IL-4 alone cannot generate LAK activity from PBMC but can enhance specific CTL generation. IL-4 inhibited LAK induction by IL-2 but enhanced CTL induction by IL-2 (Spits et al. (1988) J. Immunol. 141:29; Brooks and Rees, (1988) Clin. Exp. Immunol. 74:162). Recently, it was reported that IL-4 enhanced cellular proliferation of IL-2 preactivated cells (Kawakami et al. (1989) J. Immunol. 142: 3452).

SUMMARY OF THE INVENTION

The present invention provides an improved process for preparing adherent lymphokine-activated killer (A-LAX) cells. The process comprises:

a) treating PBMC with a lower alkyl amino acid ester, preferably PME, to deplete the monocytes;

b) culturing the remaining cells at $5\times10^6$ to $1\times10^7$ cells/mL in culture medium containing IL-2 in a plastic container to which a portion of the LAK cells adhere;

c) removing the nonadherent cells; and

d) culturing A-LAK cells in medium containing IL-2 and IL-4 to expand the A-LAK cells. The improvement comprises the last step of enhancing A-LAK cell proliferation and cytolytic activity by addition of IL-4 after the cells are adherent to the plastic surface of the container.

This invention also features a composition of the isolated A-LAK cells prepared by the above process, and a method of treating a tumor in mammal by administering the composition with IL-2.

DETAILED DESCRIPTION OF THE INVENTION

The suspension of peripheral blood mononuclear cells (PBMC) or peripheral blood lymphocytes (PBL) is cultured for an incubation period of about 2 to 21 days at 35° to 39°C, preferably 37°C, in presence of about 4 to 7% $CO_2$. Culturing is carried out at a cell concentration in the range of about $1 \times 10^6$ to $2 \times 10^7$, preferably $5 \times 106$ to $1 \times 10^7$, cells per mL, in medium containing IL-2 in concentration of about 150 to 2000 pM, preferably 1000 to 2000 pM. Culturing can be performed in conventional containers, such as T-flasks, but is preferably performed in closed, gas permeable sterile bags such as SteriCell™ cell culture bags (E. I. Du Pont de Nemours & Co., Wilmington, DE). Culturing under these conditions generates LAK cells, a population of cytolytic cells able to lyse tumor cells which are resistant to lysis by NK cells.

LAK cells prepared by this invention are used in adoptive immunotherapy in the manner described in U.S. Patent 4,849,329 to Leung and Rinehart, issued 2/3/89, which is incorporated herein by reference.

The preferred L-amino acid is phenylalanine or tyrosine and most preferred is phenylalanine. The lower alkyl group of the ester can be methyl, ethyl, propyl, isopropyl, butyl, isobutyl or t-butyl but is preferably methyl or ethyl and is most preferably methyl. Preferred pharmaceutically acceptable salts are the hydrogen chloride and hydrogen bromide salts.

The use of PME and other lower alkyl amino acid esters in a process to prepare LAK cells is disclosed in and U.S. Patent 4,849,329. Also incorporated herein by reference is U.S. patent application Serial No. 07/313,421, filed February 21, 1989.

We have previously employed PME at concentrations of about 1 to 5 mM as a single step for monocyte depletion in a process to generate A-LAK cells. The process is disclosed in coassigned U.S. Patent Application Serial No. 07/384134, filed July 21, 1989, which is incorporated herein by reference. It was discovered that it is possible to generate A-LAK from PME-treated PBMC and that treatment of PBMC with PME prior to culturing the A-LAK cells in plastic containers, results in a substantial increase in the level of expansion of the A-LAK cells, relative to the expansion in cell number obtained in the absence of PME treatment. During the expansion of the A-LAK cells, following treatment with PME, the LAK cell functional cytolytic activity remains high.

The present invention involves an improved process for the expansion and enrichment of Leu19[+] lymphocytes with high levels of LAK cell lytic activity. PBMC are first treated with PME to remove monocytes and other PME-sensitive cells. The resulting lymphocytes are then incubated with IL-2 in plastic flasks for 1 to 2 days. The adherent cells are then cultured with IL-4 for 8 to 21 days to obtain an increase in cell number and LAK activity. The cell densities used during the A-LAK preparation process, including the monocyte depletion step, are preferably about $5 \times 10^6$ to $2 \times 10^7$ cells/mL, more preferably about $1 \times 10^7$ cells/mL.

Our procedure using IL-4 provides several substantial differences and important advantages over the procedures previously used to prepare A-LAK cells (Melder et al. (1988) Cancer Research 49: 144; our patent application USSN 07/384,134). First, use of PME as a chemical agent to deplete monocytes from PBMC allows the process to be better controlled by PME concentration and time of incubation than can be achieved using the nylon-wool procedure. PME treatment of PBMC is also much more convenient and less time consuming to use than centrifugal elutriation which requires an elaborate setup, sterilization procedure, and the running of the elutriation. Second, in the process of the invention, the lymphocytes are cultured in plastic containers at a cell density of about 1 to $2 \times 10^7$/mL, as compared with $2 \times 10^6$/mL which has been used previously. Thus, the present process will reduce the total volume of medium used by 5 to 10-fold, relative to previous procedures for the preparation of A-LAK cells. Third, the high cell density ($1 \times 10^7$/mL) generally gave more reliable expansion than low cell density ($1 \times 10^6$/mL). Fourth, enriched A-LAK cells can be generated from PBMC completely depleted of monocytes, but they are not as proliferative. The amount of monocyte depletion can be precisely controlled by the PME concentration used to treat the PBMC. As described above, the level of monocyte depletion cannot be readily controlled using elutriation and/or the nylon-wool column method of depleting monocytes. Fifth, the inclusion of IL-4 further enhanced A-LAK cellular expansion and proliferation and LAK cytolytic activity.

The A-LAK procedure of the invention, which includes the use of IL-4, provides an improvement relative to than the conventional method of generation LAK cells, such as that described by Kawakami et al. (1989) J. Immunol. 142: 3452. Our method using high cell density adherence and IL-4 gave good enrichment and cellular expansion of the A-LAK cells.

In the Examples which follow, a 3-hour $^{51}$Cr-release assay was used to measure cytotoxicity of LAK cells for tumor cells. Tumor cells, at a concentration of about $2x10^6$ to $10x10^6$, were incubated with 50 $\mu$Ci of Na2[51]CrO$_4$ in 0.4 mL of Tris-phosphate buffered saline for 1 hour at 37°C. The cells were washed 4 times with RPMI 1640 containing 10% fetal calf serum (FCS) and resuspended to $10^5$ cells/mL in RPMI 10% FCS. The effector cells (LAK cells) were suspended to various concentrations and 0.1 mL was added to wells in round bottom microtiter plates. The $^{51}$Cr-labeled target cells (0.1 mL) are added to all wells and the plates were centrifuged at 200 xg for 5 minutes. After 4 hours of incubation at 37°C, the plates are centrifuged again and 0.1 mL of resulting supernatant was removed from each well and counted in a gamma counter. Percent cytotoxicity was calculated from the following formula:

$$\% \text{ cytotoxicity} = \frac{\text{experimental cpm} - \text{spontaneous cpm} \times 100}{\text{total cpm} - \text{spontaneous cpm}}$$

Each variable was tested in triplicate and the resulting data expressed as % cytotoxicity or lysis. This cytotoxicity test is further described in Selected Methods in Cellular Immunology, Mishell and Shiigi, eds., 124-137, W. M. Freeman and Co., San Francisco (1980).

For cell surface marker analysis, $2X10^5$ cells in 0.1 mL of chilled staining buffer solution (PBS, 15% BSA, and 0.1% sodium azide) were placed in 96-well round bottom plate. Various fluorescein-tagged antibodies were added to the cells for 30 minutes at 4°C. The cells were washed twice and resuspended in 1% paraformaldehyde prior to analysis for florescence on a flow cytometer (Becton-Dickinson, Mountain View, CA). Table 1 summarizes the reactivity/specificity of the antibodies used in this study.

Table 1

| Differentiation Antigens Identified by Monoclonal Antibodies (mAb) in this Study | | |
|---|---|---|
| CD Cluster | mAb | Cellular Reactivity/Specificity |
| CD3 | Leu4 | T cells |
| CD4 | Leu3 | Helper/inducer T cell subset |
| CD8 | Leu2 | T cell subset, NK cell subset |
| CD56 | Leu19 | NK cells, LAK cells |

Example 1

Effect of IL-4 on A-LAK Cell Generation

PBMC were treated with 5 mM PME to deplete monocytes and cultured at $5x10^6$ cells/mL with 10 U/mL IL-2, or IL-2 and IL-4, for 20 hours in plastic flasks. Nonadherent (NA) cells were removed and the cells adherent to flasks were cultured with media containing IL-2, or IL-2 and IL-4. IL-4 (Genzyme, Boston, MA) was added to the adherent cells generated in media with IL-2 alone. NA cells were cultured similarly. The A-LAK cells expanded 123-fold when cultured with IL-2 alone in 14 days as shown in Table 2.

## Table 2

### Effect of IL-4 on A-LAK Cell Expansion, Cytotoxicity and Phenotype

| IL-4 (mL) | Fold Expansion | % Lysis | % Leu 2 | % Leu 3 | % Leu 4 | % Leu 19 |
|---|---|---|---|---|---|---|
| **After Adherence** | | | | | | |
| 0 | 123 | 80 | 58 | 4 | 8 | 99 |
| 10 | 146 | 78 | 57 | 3 | 5 | 99 |
| 50 | 412 | 76 | 57 | 2 | 7 | 99 |
| 200 | 547 | 82 | 50 | 2 | 4 | 99 |
| **During Adherence** | | | | | | |
| 0 | 123 | 80 | 58 | 4 | 8 | 99 |
| 10 | 46 | 69 | 49 | 21 | 27 | 76 |
| 50 | 40 | 56 | 35 | 65 | 77 | 27 |
| 200 | 30 | 43 | 30 | 77 | 88 | 17 |

PBMC were treated with 5 mM PME to deplete monocytes and cultured at $5 \times 10^6$ cells/ml with 10 U/mL IL-2 or IL-2 and IL-4 for 20 hr in plastic flasks. Nonadherent cells were removed and the cells adherent to flasks were cultured with media containing IL-2 or IL-2 and IL-4. IL-4 was added to the adherent cells generated in media with IL-2 alone. Data shown were at 14 days of culture. Cytotoxicity shown was at E:T ratio of 5:1 against 51Cr-labeled Raji target cells in a 3 hr assay. IL-4 added after cells adhered gave four times greater cellular expansion than IL-2 alone. On the other hand, cellular expansion was suppressed by IL-4 when it was present during the adherent phase, although there was still a 30- to 46- fold cellular expansion.

A-LAK cells generated with IL-2 had 80% specific lysis of Raji target cells at an effector to target ratio of 5:1 in a 3 hour $^{51}$Cr-release assay. When IL-4 was added after the adherence phase, it had no inhibitory effect on the LAK activity. However, when IL-4 was present during the adherence phase it inhibited the LAK activity induced by IL-2.

Phenotyping of cells grown in media containing IL-2 and IL-4 revealed that IL-4 inhibited the percent Leu19[+] cells from 99% to 17% as shown in Table 2. At the same time, Leu3 and Leu4 cells were increased to 77 to 88% from about 4-8% in the absence of IL-4, with IL-2 alone. Therefore, IL-4 preferentially inhibited Leu19[+] cellular activating and proliferation induced by IL-2, when it was present during the adherence phase of the process. It is also possible that Leu19[+] cells had a negative effect on the proliferation of Leu3[+] cells and inhibition of Leu19[+] cells by IL-4 thus allowed the Leu3[+] cells to proliferate in response to IL-2 and/or IL-4. When IL-4 was added after the adherence phase, it had no inhibitory effect on the percent Leu19[+] cells as shown in Table 2.

In summary, IL-4 inhibited A-LAK generation when it was present during the adherence phase of the process and enhanced a population of cells enriched in CD4 (Leu4[+]). On the other hand, IL-4 enhanced the cellular expansion and the cytotoxicity of A-LAK cells when it was present after the adherence phase of the A-LAK generation process.

### Example 2

PBMC were treated with 5 mM PME for 40 min to deplete monocytes and incubated with 10 U/mL of IL-2 for 1 day. Cells adherent to the plastic were cultured with IL-2, or IL-2 plus IL-4. A-LAK cells were expanded during the culture periods of 8, 11, 15 days. As shown in Table 3, IL-4 enhanced the cellular expansion of A-LAK cells. The percent of Leu19[+] cells and the cytotoxicity against the Raji target cells were not diminished in the presence of IL-4.

Table 3

| | | Total Cells (x10$^6$) | % Leu3 (CD4) | % Leu19 (CD56) | % Lysis E:T Ratio | |
|---|---|---|---|---|---|---|
| Effect of IL-4 on A-LAK Cells When IL-4 is Added After the Adherence Phase | | | | | | |
| | | | | | 2.5:1 | 1.25:1 |
| Day 0 | Control<br>IL-4 | 1.9<br>1.8 | | | | |
| Day 8 | Control<br>IL-4 | 42<br>78 | 8<br>8 | 95<br>98 | 85<br>85 | 75<br>68 |
| Day 11 | Control<br>IL-4 | 90<br>324 | ND<br>ND | ND<br>ND | 94<br>89 | 84<br>90 |
| Day 15 | Control<br>IL-4 | 252<br>1200 | 4<br>2 | 98<br>99 | 70<br>70 | 56<br>65 |

Adherent cells were generated from PME-treated PBMC at 5x10$^5$ cells/mL with 10 U/mL IL-2 for 1 day. The adherent cells were then cultured with IL-2 (control), or IL-2 and IL-4 (200 U/mL), for the various time periods.

Example 3

Adherent cells were generated with 0.1, 1, or 10 U/mL of IL-2 for 1 day before the IL-4 addition. The adherent cells were cultured for an additional 7 days. IL-4 enhanced the cellular expansion and LAK lytic activity against the Raji target cells (Table 4).

<u>Table 4</u>

Effect of Dose of IL-2 on IL-4

Modulation of A-LAK Cells

| IL-2 U/mL | | Total Cells (X10^6) | | % Leu 3 | % Leu 19 | % Lysis |
|---|---|---|---|---|---|---|
| **Day 8** | | | | | | |
| 0.1 | Control | 6 | | 20 | 68 | 16.3 |
| | IL-4 | 6 | | 27 | 48 | 16.0 |
| 1 | Control | 26 | | 7 | 88 | 74.8 |
| | IL-4 | 24 | | 12 | 79 | 73.8 |
| 10 | Control | 40 | | 8 | 87 | 85.2 |
| | IL-4 | 51 | | 8 | 93 | 76.7 |
| **Day 13** | | | | | | |
| 0.1 | Control | 12 | | 59 | 37 | 17.1 |
| | IL-4 | 19 | | 79 | 18 | 12.0 |
| 1 | Control | 110 | | 55 | 44 | 43.5 |
| | IL-4 | 180 | | 32 | 58 | 87.4 |
| 10 | Control | 400 | | 5 | 93 | 86.1 |
| | IL-4 | 516 | | 5 | 93 | 94.6 |

Adherent cells were generated from PME-treated PBMC at $1 \times 10^7$ cells/mL with 0.1, 1 or 10 U/mL IL-2 for 1 day. The adherent cells were then cultured with IL-2 alone (control), or IL-2 and IL-4 (200 U/mL), for the various time periods.

The $^3$H-thymidine incorporation was also enhanced in the IL-4 groups as shown in Table 5.

### Table 5
### Effect of IL-4 on A-LAK Cellular Proliferation

#### IL-2, (U/mL)

| | 0.1 | 1 | 10 |
|---|---|---|---|
| **Day 8** | | | |
| Control | 1547 | 6924 | 20730 |
| L-4 | 1884 | 8957 | 27502 |
| **Day 13** | | | |
| Control | 1470 | 11340 | 21307 |
| IL-4 | 4670 | 22135 | 33469 |

Adherent cells were generated from PME-treated PBMC at $5 \times 10^6$ cells/mL with 0.1, 1, or 10 U/mL IL-2 for 1 day. The adherent cells were then cultured with IL-2 (control), or IL-2 plus IL-4 (200 U/mL), for the various time periods.

Example 4

Nonadherent LAK (NA-LAK) cellular expansion was somewhat enhanced by IL-4 when it was added after IL-2 activation and after the adherence of the A-LAK population as shown in Table 6. The LAK activity remained the same relative to the use of IL-2 alone. However, when IL-4 was present at the same time as IL-2 (during the adherence phase of the A-LAK cells), LAK activity was inhibited as shown in Table 6. The cellular expansion was about the same with IL-2 alone compared to IL-2 plus IL-4, when IL-4 was present during the adherence step. Analysis of the surface markers of the cells showed only a slight increase in CD4[+] cells when IL-4 was present during the IL-2 activation as shown in Table 6.

### Table 6
### Effect of IL-4 on NA-LAK Cell Expansion,
### Cytotoxicity and Phenotype

| IL-4 (mL) | Fold Expansion | % Lysis | % Leu 2 | % Leu 3 | % Leu 4 | % Leu 19 |
|---|---|---|---|---|---|---|
| **After Adherence** | | | | | | |
| 0 | 2.8 | 73 | 58 | 23 | 32 | 72 |
| 10 | 4.2 | 78 | 54 | 18 | 29 | 76 |
| 50 | 5.2 | 71 | 49 | 18 | 25 | 78 |
| 200 | 5.2 | 68 | 56 | 17 | 24 | 82 |
| **During Adherence** | | | | | | |
| 0 | 2.8 | 73 | 58 | 23 | 32 | 72 |
| 10 | 2.3 | 64 | 62 | 22 | 34 | 70 |
| 50 | 2.1 | 54 | 59 | 32 | 46 | 56 |
| 200 | 2.0 | 56 | 53 | 34 | 47 | 55 |

Therefore, although the effects of IL-4 on LAK expansion or CD4[+] expansion could also be observed with the NA-LAK cell cultures, the enhanced proliferation of cells was substantially reduced compared with the adherent cell (A-LAK) cultures.

**Claims**

1.  A process for preparing adherent lymphokine-activated killer (A-LAK) cells, comprising:
    a) treating PBMC with a lower alkyl amino acid ester to deplete the monocytes;
    b) culturing the remaining cells at $5 \times 10^6$ to $1 \times 10^7$ cells/mL in culture medium containing IL-2 in a plastic container to which a portion of the LAK cells adhere;
    c) removing the nonadherent cells; and
    d) culturing A-LAK cells in medium containing IL-2 and IL-4 to expand the A-LAK cells.

2.  A composition comprising isolated A-LAK cells prepared by the process of claim 1, said A-LAK cells being in a pharmaceutically acceptable carrier and being reactive to a tumor when administered with IL-2 to a mammal afflicted with the tumor.

3.  A method of treating a tumor in a mammal comprising administering to the mammal IL-2 and a tumor-inhibiting amount of a composition according to claim 2.